# EUROPEAN PATENT SPECIFICATION

(11) **EP 1 329 438 B1**
(45) Date of publication and mention of the grant of the patent: **08.04.2009**
(21) Application number: 03447009.6
(22) Date of filing: 14.01.2003
(51) Int. Cl.: C04B 35/634, C04B 38/00, B22F 1/00

(54) **Method for producing metallic and ceramic products**
Verfahren zur Herstellung von metallischen und keramischen Produkten
Procédé de fabrication de produits métalliques et céramiques

(30) Priority: 26.04.2002 EP 02447076; 14.01.2002 EP 02447006
(43) Date of publication of application: 23.07.2003
(73) Proprietor: "VLAAMSE INSTELLING VOOR TECHNOLOGISCH ONDERZOEK", afgekort "V.I.T.O.", 2400 Mol (BE)
(72) Inventor: Cooymans, Jozef, 2400 Mol (BE); De Wilde, Anne-Marie, 2400 Mol (BE); Thijs, Ivo, 2400 Mol (BE); Mullens, Steven, 3740 Bilzen (BE); Snijkers, Frans, 3930 Hamont-Achel (BE); Luyten, Jan, 3294 Diest (BE)
(74) Representative: pronovem

(56) References cited:
- EP-A- 0 488 060
- EP-A- 1 018 495
- WO-A-02/16285
- US-A- 5 665 440
- US-A- 5 697 043
- US-A- 5 770 136

## Description

### Field of the invention

The present invention is related to a method for producing metallic and ceramic products. More particularly, the present invention is related to the use of natural gel forming compounds in a method for producing metallic or ceramic products, advantageously for producing metallic or ceramic foams having a controlled cell structure using hollow shapes.

### State of the art

Various production routes for ceramic and metallic foam structures exist. The main characteristics like pore size distribution, relative density, cell morphology and strut density determine the ultimate properties of the foam and thus the field of application. Due to the wide range of application for such materials, several manufacturing routes have been proposed. The major fabrication route is the polyurethane replica technique, where reticulated polyurethane foam is coated with ceramic slurry.

However, the major difficulty with the above mentioned technique exists in a strict control of both the macro- and microstructure of the foam, accompanied with sufficient mechanical strength. For example, the polyurethane replica technique provides foam structures with a well-controlled pore size distribution and strut thickness (owing to a strict process control for the basic material, namely the polyurethane foam). However, as the polymeric foam is calcinated, the strut becomes hollow, giving rise to poor mechanical strength. Other techniques lead to dense struts and strong porous material, but the control over the macro- and microstructure is much more difficult.

As the simultaneous existence of both requirements is essential for the bulk of the applications for ceramic or metallic porous materials, the developments are focussing on new and improved manufacturing routes.

Metallic and ceramic foams are porous materials with very low density (5 to 30% of theoretical density) which are deployed in diverse applications such as filters for molten metals, oven or furnace walls, soot filters, carriers for catalysts, biomedical implants, electro-ceramics, ... . Useful implementations of metallic or ceramic foams requires foams with sufficient strength, which have a controlled structure on both micro and macro level.

Several methods for making such metallic or ceramic foams have been described, however none of these methods provide the superior properties needed for some applications.

Methods for making ceramic foams using hollow spheres have been described by D.J. Green, "Fabrication and mechanical properties of lightweight ceramics produced by sintering of hollow spheres", J.Amer.Ceram.Soc. Vol 68,no7, pp 403-409 and by U. Waag et al. in "Metallic hollow spheres - materials for the future", MPR January 2000, pp 29-33.

Document US-A-5 665 440 discloses a method for producing ceramic hollow shapes using a support material that is not sacrificial, i.e. it is not burned off, but removed.

### Aims of the invention

The present invention aims to provide a new method for producing metallic and ceramic products and in particular for providing a new method for producing metallic and ceramic foams. A further aim of the invention is to provide a method for producing strong, closed and/or open metallic and ceramic foams. The new method should provide a precise control on the main characteristics of the product, together with good mechanical strength. Further, the present invention aims at avoiding toxicity and pollution issues as present in the current state of the art methods.

### General Description of the invention

The present invention a method for producing ceramic hollow shapes, comprising the following steps:
- Preparation of a stable ceramic powder slurry comprising a gelling agent, with predefined rheological properties,
- Providing sacrificial support material,
- Coating said support material with said ceramic slurry,
- A drying step, and
- An optional burning step and/or a presintering step depending on the sacrificial support material..

Said gelling agent is preferably a biogel former, advantageously selected from the group consisting of gelatine, ovalbumin, agar, carageenan, inulin, pectine, starch, potato dextrin, guar, caseinate, gellan, alginate, locust bean gum, xanthan and carboxy-methyl-cellulose.

Further, this method can be followed the following steps to obtain a foam structure:
- Arranging hollow shapes as obtained by the method as described higher in a matrix,
- A fixing step wherein said hollow shapes are interconnected by addition of ceramic slurry as above, followed by a drying step.

Preferably, the method further comprising a burning step, a presintering step and a sintering step.

The predefined rheological properties preferably comprise a viscosity lower than 0.01 Pas. The sacrificial support material preferably has dimensions between 20 µm and 5 mm. The method according to this embodiment of the present invention can comprise a burning out step which is advantageously performed at a temperature between 500 and 600°C. The method can also comprise a presintering step which can be performed at a temperature between 1000 and 1200°C.

In the method according to this first embodiment of the present invention, the form factor of the sacrificial support material can selected from the group consisting of spherical, cubical, ellipsoid, cylindrical, tubular, pyramidal and oblong. Any shape is possible.

In a preferable embodiment of the invention, the method further comprises a repetition of the coating steps prior to arranging the hollow shapes in a matrix. This way, the mechanical strength of the hollow shapes and thus also of the foams obtained therewith can be increased. Also preferable is to add a fugitive phase to the stable ceramic powder slurry. This way, an open cell structure can be obtained.

The arranging step can comprise arranging hollow shapes of predetermined dimensions at predetermined positions in said matrix. Also, when hollow shapes of different sizes are arranged to form a gradient, a foam structure gradient is obtained.

Another aspect of the present invention is a green ceramic or metallic product obtainable by any of the methods of the present invention and comprising a biogel former. A green ceramic or metallic product is an intermediate product (before calcination and sintering).

### Short description of the figures

Figure 1 a and b show respectively a ceramic foam produced starting from styrene granules and a ceramic foam produced starting from peas and seeds having a different diameter.

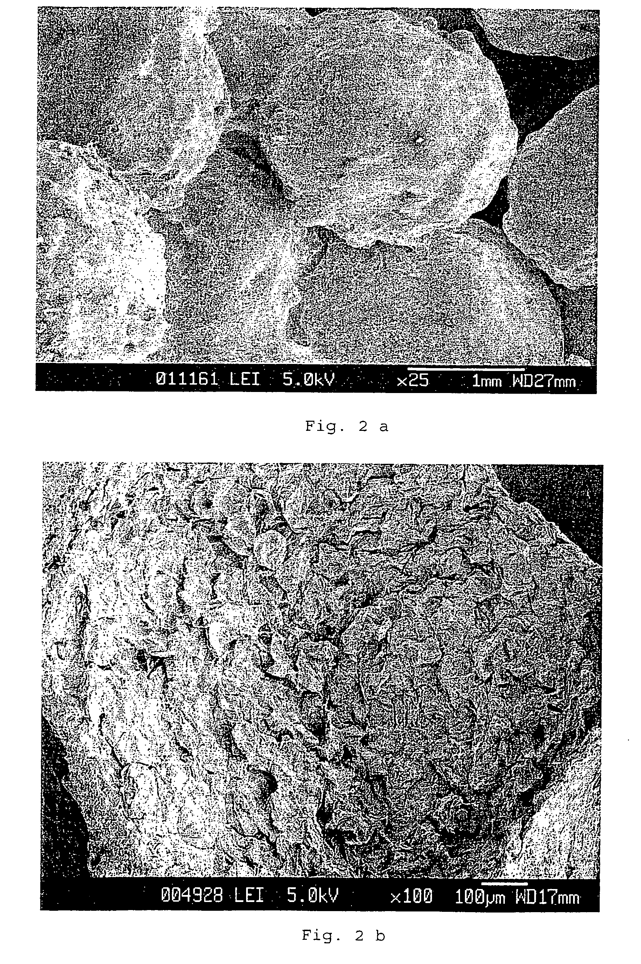

Figure 2 a and b show respectively the structure of the hollow spheres and the surface roughness.

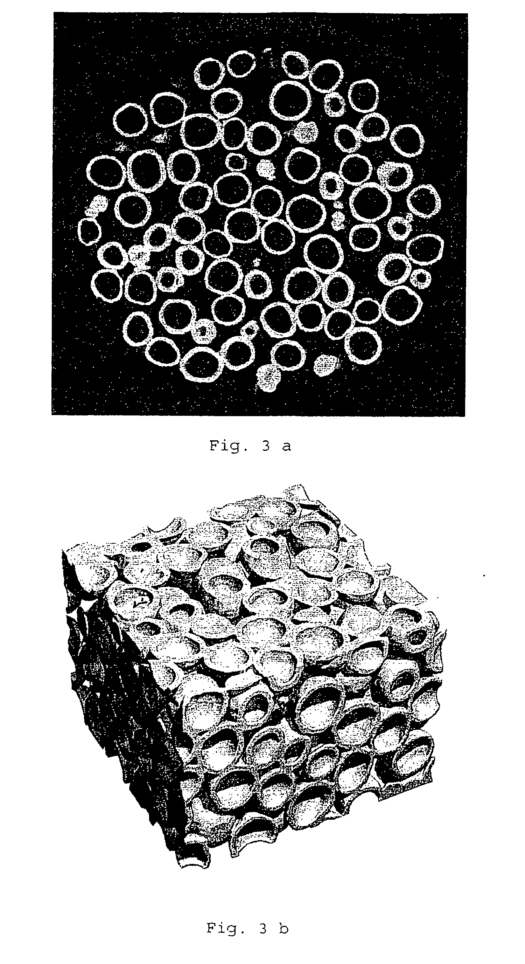

Figure 3 shows an X-ray tomographic analysis of ceramic foam manufactured by the hollow spheres method: (a) two dimensional cross section and (b) multislice reconstruction into three dimensional structure.

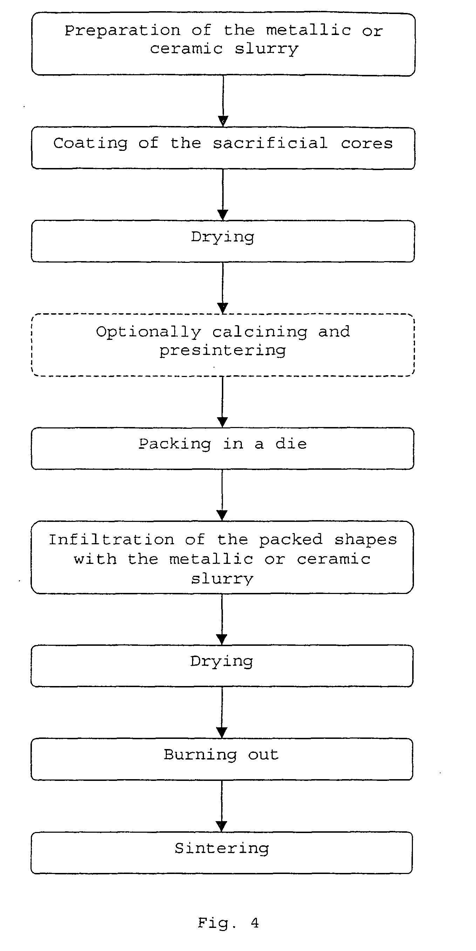

Figure 4 shows a flowchart of the method for producing metallic or ceramic hollow shapes and foams according to the present invention.

### Detailed description of the invention

The present invention concerns the use of natural compounds such as biogel formers (also called hydrocolloid or biobinder) as a binder. Preferably, water-based suspensions are used for this, as the natural compounds can readily dissolve in water. Typical examples of such biogel forming compounds are gelatin, ovalbumin, agar, carageenan, ... .

The present invention comprises in a first embodiment a method for producing ceramic hollow spheres and strong, open and/or closed ceramic foams built from said hollow shapes. The metallic and ceramic hollow shapes produced according to the invention preferably have a predetermined size range of 20 µm to 5 mm. The method according to the invention comprises the use of sacrificial support material such as styrene, seeds, nuts, peas, ... . The method also comprises the use of a metallic or ceramic slurry, preferably comprising a natural gel-forming compound such as gelatine, agar, carrageen, ... .

The method for producing metallic or ceramic hollow shapes more particularly comprises the following steps:
- Preparation of a stable metallic or ceramic powder slurry comprising a gelling agent, with predefined rheological properties (viscosity preferably lower then 0.01 Pas),
- Providing sacrificial support material (such as styrene, seeds, nuts, peas, ...),
- Coating said support material with said metallic or ceramic slurry,
- A drying step, and
- An optional burning out step (e.g. 2 hours at a temperature between 500 and 600 °C) depending on the sacrificial support material (if it needs to be burned off).

The metallic or ceramic slurry thus comprises preferably water (as alternative, a solvent can be used), metallic or ceramic powder, dispersant and a bio-gelling agent. Other additives can be added. The coating step can be performed using a spray dry set-up and/or by rolling the support material in a cylinder filled with said metallic or ceramic slurry. The temperature has to be higher than the gelling temperature of the gelling agent. The precise formulation depends on the weight of the support material shapes. The bio-gelling agent provides for wetting the support material surface and by gelling after cooling down for good adhesion.

The method according to the invention for producing metallic or ceramic foams built from hollow shapes further comprises the following steps:
- Arranging hollow shapes as obtained by the method of the invention in a matrix,
- A fixing step wherein said hollow shapes are interconnected by addition of metallic or ceramic slurry as above, followed by a drying step.

The method preferably further comprises a burning out step (500-600°C), and for ceramics a presintering step (1000-1200°C) and a sintering step (1400-1700°C). For metals the sintering temperatures are much lower, starting at about 500°C. The exact temperatures for these different heat treatments depend on the kind of core material and of the metallic or ceramic powder used.

Many uses of the metallic and ceramic materials according to the invention can be imagined. Metallic or ceramic foams created according to the present invention with a tailored cell structure find applications as new thermal isolation material, sensors and actuators, biomaterials and so on.

### Examples

### Example 1

As the sacrificial core, styrene granules with mean size 2 mm are used. The ceramic slurry consists of Al₂O₃ powder, CT3000SG (Alcoa) 15 vol%, a bio-binder e.g. gelatine (1 to 5 wt%/water), water and Darvan C. The viscosity has to be lower than 0.01 Pas

The styrene granules are coated by using a combination of spray drying and turning vessel set up.

The coated styrene granules are packed in a mould and infiltrated with the same ceramic slurry as for the first coating. Hereby the neck formation between the hollow shapes is realised. The heat treatments are as follows:
- Drying at room temperature
- Burning out 2 hours at 600°C
- Presintering 2 hours at 1100°C
- Sintering 1 hour at 1650°C

The compaction strength of the hollow shapes produced from the styrene granules is 22N for a layer thickness of 0.3mm.

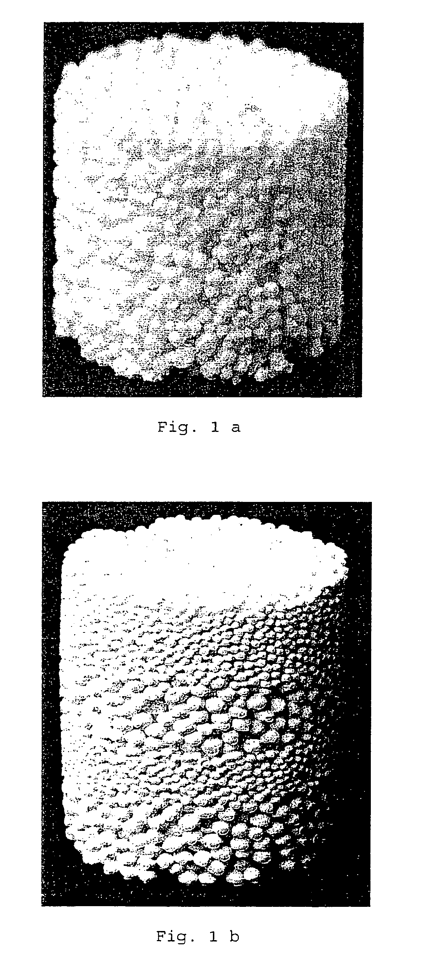

The compaction strength of cylinders built up with these hollow elements is 2.5 MPa for a density of 15-16% (of the theoretical density). The result can be seen in figure 1 a.

### Example 2

- Sacrificial core: peas (diameter 4 mm).
- Slurry idem as in example 1.
- The coating of the peas was done in a turning vessel.
- Packing, second coating and the heat treatment were done as in example 1.

The mechanical compaction strength of the spheres is 22N for a layer thickness of 0.3mm. The mean compaction strength of cylinders built up with this material is 0.9 MPa for a density of 11-12 %.

### Example 3

- Sacrificial core: seed (diameter 2 mm.)
- Slurry idem as in example 1
- Coating, packing, second coating and heat treatment as in example 2

The mean compaction strength of the obtained hollow spheres is 31 N for a layer thickness of 0.3 mm. The mean compaction strength of cylinders built up with these hollow spheres is 4 MPa for a density of 22.5%.

### Example 4

- Coating of seeds and peas is done as in examples 2 and 3.
- Packing is done by providing alternating layers of coated peas and coated seeds.
- Second coating and heat treatment as in example 2.

The result can be seen in figure 1 b. The alternating layers of seeds 1 and peas 2 are clearly visible.

## Claims

1. A method for producing metallic or ceramic hollow shapes, comprising the following steps:
(i) preparation of a stable metallic or ceramic powder slurry comprising a gelling agent, with a viscosity lower than 0.01 Pas,
(ii) providing sacrificial support material,
(iii) coating said support material with said metallic or ceramic slurry,
(iv) a drying step, and
(v) a burning out step.

2. The method as in claim 1, further comprising a presintering step.

3. Method as in claim 1 or 2, **characterised in that** said gelling agent is a biogel former selected from the group consisting of gelatine, ovalbumin, agar, carageenan, insulin, pectine, starch, potato dextrin, guar, caseinate, gellan, alginate, locust bean gum, xanthan, carboxy-methyl-cellulose.

4. A The method according to claim 1 or 2, for producing metallic or ceramic foams built from hollow shapes, further comprising the following steps:
• arranging hollow shapes as obtained by the method of claim 1 or 2 in a matrix,
• a fixing step wherein said hollow shapes are interconnected by addition of the metallic or ceramic slurry of as in claim 1, followed by a drying step.

5. The method as in claim 4, further comprising a burning step, a presintering step and a sintering step.

6. A The method according to claim 1, for producing metallic or ceramic foams, comprising:
• performing the steps (i) to (iv) of claim 1 to obtain a coated and dried support material and further comprising the following steps:,
• arranging said coated and dried support material in a matrix,
• a fixing step wherein the support material is interconnected by addition of the metallic or ceramic slurry of as in claim 1, followed by
• a drying step,
• performing step (v) of claim la burning out step and
• a sintering step.

7. The method as in any of the claims 1 to 3 and 6, wherein the sacrificial support material has dimensions between 20 µm and 5 mm.

8. The method as in any of the claims 1 to 3, 6 and 7, wherein the form factor of the sacrificial support material is selected from the group consisting of spherical, cubical, ellipsoid, cylindrical, tubular, pyramidal and oblong.

9. The method of claim 4 or 5, further comprising a repetition of the steps of claim 1 prior to any of the steps of claim 4.

10. The method as in any of the claims 1 to 9, wherein the stable metallic or ceramic powder slurry further comprises a fugitive phase.

11. The method as in any of the claims 4 to 9, wherein the arranging step comprises arranging hollow shapes of different sizes to form a gradient.

## Patentansprüche

1. Verfahren zur Herstellung von metallischen oder keramischen Hohlformen, umfassend die folgenden Schritte:
(i) die Vorbereitung einer beständigen Suspension aus Metall- oder Keramikpulver, umfassend ein Geliermittel mit einer Viskosität von weniger als 0,01 Pas,
(ii) die Bereitstellung eines Opferträgermaterials,
(iii) die Beschichtung des Trägermaterials mit der Metall- oder Keramiksuspension,
(iv) einen Trocknungsschritt und
(v) einen Ausbrennschritt.

2. Verfahren nach Anspruch 1, ferner umfassend einen Vorsinterschritt.

3. Verfahren nach Anspruch 1 oder 2, **dadurch gekennzeichnet, dass** das Geliermittel ein Biogelformer ist, das aus der Gruppe gewählt wird, die aus Gelatine, Ovalbumin, Agar-Agar, Carrageen, Inulin, Pektin, Stärke, Kartoffeldextrin, Guar, Kaseinat, Gellan, Alginat, Johannisbrotgummi, Xanthan, Carboxymethylcellulose besteht.

4. Verfahren nach Anspruch 1 oder 2 zur Herstellung von Metall- oder Keramikschaumstoffen, die aus Hohlformen aufgebaut werden, ferner umfassend die folgenden Schritte:
- die Anordnung von Hohlformen, wie sie durch das verfahren nach Anspruch 1 oder 2 erzielt werden, in einer Matrix,
- einen Fixierschritt, wobei die Hohlformen untereinander verbunden werden durch Hinzufügen der Metall- oder Keramiksuspension nach Anspruch 1, gefolgt von einem Trocknungsschritt.

5. Verfahren nach Anspruch 4, ferner umfassend einen Brennschritt, einen Vorsinterschritt und einen Sinterschritt.

6. Verfahren nach Anspruch 1 zur Herstellung von Metall- oder Keramikschaumstoffen, umfassend:
- die Ausführung der Schritte (i) bis (iv) nach Anspruch 1, um ein beschichtetes und getrocknetes Trägermaterial zu erzielen, und ferner umfassend die folgenden Schritte:
- die Anordnung des beschichteten und getrockneten Trägermaterials in einer Matrix,
- einen Fixierschritt, wobei das Trägermaterial untereinander verbunden wird durch das Hinzufügen der Metall- oder Keramiksuspension nach Anspruch 1, gefolgt von
- einem Trocknungsschritt,
- der Ausführung von Schritt (v) nach Anspruch 1, einem Ausbrennschritt und
- einem Sinterschritt.

7. Verfahren nach einem der Ansprüche 1 bis 3 und 6, wobei das Opferträgermaterial Abmessungen zwischen 20 µs und 5 mm aufweist.

8. Verfahren nach einem der Ansprüche 1 bis 3, 6 und 7, wobei der Formfaktor des Opferträgermaterials aus der Gruppe ausgewählt wird, die aus sphärisch, kubisch, ellipsoidisch, zylindrisch, röhrenförmig, pyramidenförmig und rechteckig besteht.

9. Verfahren nach Anspruch 4 oder 5, ferner umfassend eine Wiederholung der Schritte nach Anspruch 1 vor einem der Schritte nach Anspruch 4.

10. Verfahren nach einem der Ansprüche 1 bis 9, wobei die beständige Suspension aus Metall- oder Keramikpulver ferner eine flüchtige Phase umfasst.

11. Verfahren nach einem der Ansprüche 4 bis 9, wobei der Anordnungsschritt das Anordnen von verschieden großen Hohlformen zum Bilden eines Gradienten umfasst.

## Revendications

1. Procédé de production de formes creuses métalliques ou céramiques, comprenant les étapes suivantes :
(i) la préparation d'une bouillie stable de poudre de métal ou de céramique comprenant un agent gélifiant, d'une viscosité inférieure à 0,01 Pas,
(ii) la fourniture d'un matériau de support sacrificiel,
(iii) le couchage dudit matériau de support avec ladite bouillie métallique ou céramique,
(iv) une étape de séchage, et
(v) une étape de cuisson.

2. Procédé selon la revendication 1, comprenant en outre une étape de préfrittage.

3. Procédé selon la revendication 1 ou 2, **caractérisé en ce que** ledit agent gélifiant est un formateur de biogel sélectionné dans le groupe composé de la gélatine, de l'ovalbumine, de l'agar-agar, de la carraghénane, de l'inuline, de la pectine, de l'amidon, de la dextrine de pomme de terre, de guar, du caséinate, de la gellane, de l'alginate, de la gomme de caroube, du xanthane, de la carboxyméthylcellulose.

4. Procédé selon la revendication 1 ou 2, pour la production de mousses métalliques ou céramiques construites à partir de formes creuses, comprenant en outre les étapes suivantes :
- l'agencement de formes creuses comme obtenues par le procédé selon la revendication 1 ou 2 en motrice,
- une étape de fixation dans laquelle lesdites formes creuses sont interconnectées par l'ajout de la bouillie métallique ou céramique selon la revendication 1, suivie d'une étape de séchage.

5. Procédé selon la revendication 4, comprenant en outre une étape de cuisson, une étape de préfrittage et une étape de frittage.

6. Procédé selon la revendication 1, pour la production de mousses métalliques ou céramiques, comprenant :
- l'exécution des étapes (i) à (iv) selon la revendication 1 pour obtenir un matériau de support recouvert et séché, et comprenant en outre les étapes suivantes :
- l'agencement dudit matériau de support recouvert et séché en matrice,
- une étape de fixation dans laquelle le matériau de support est interconnecté par l'ajout de la bouillie métallique ou céramique selon la revendication 1, suivie
- d'une étape de séchage,
- de l'exécution de l'étape (v) selon la revendication 1, d'une étape de cuisson et
- d'une étape de frittage.

7. Procédé selon l'une quelconque des revendications 1 à 3 et 6, dans lequel le matériau de support sacrificiel présente des dimensions entre 20 µm et 5 mm.

8. Procédé selon l'une quelconque des revendications 1 à 3, 6 et 7, dans lequel le facteur de forme du matériau de support sacrificiel est sélectionné dans le groupe composé de sphérique, cubique, ellipsoïde, cylindrique, tubulaire, pyramidal et oblong.

9. Procédé selon la revendication 4 ou 5, comprenant en outre une répétition des étapes de la revendication 1 avant l'une quelconque des étapes de la revendication 4.

10. Procédé selon l'une quelconque des revendications 1 à 9, dans lequel la bouillie stable de poudre de métal ou de céramique comprend en outre une phase volatile.

11. Procédé selon l'une quelconque des revendications 4 à 9, dans lequel l'étape d'agencement comprend l'agencement de formes creuses de différentes dimensions pour former un gradient.
